(19) Europäisches Patentamt
European Patent Office
Office européen des brevets

(11) **EP 3 167 809 A1**

(12) **EUROPEAN PATENT APPLICATION**
published in accordance with Art. 153(4) EPC

(43) Date of publication:
**17.05.2017 Bulletin 2017/20**

(51) Int Cl.:
***A61B 8/08*** *(2006.01)*

(21) Application number: **15819352.4**

(86) International application number:
**PCT/JP2015/060236**

(22) Date of filing: **31.03.2015**

(87) International publication number:
**WO 2016/006288 (14.01.2016 Gazette 2016/02)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA**

(30) Priority: **11.07.2014 JP 2014143700**

(71) Applicant: **Olympus Corporation
Tokyo 192-8507 (JP)**

(72) Inventor: **MIYAKI, Hironaka
Tokyo 192-8507 (JP)**

(74) Representative: **Gunzelmann, Rainer
Wuesthoff & Wuesthoff
Patentanwälte PartG mbB
Schweigerstraße 2
81541 München (DE)**

(54) **ULTRASONIC OBSERVATION DEVICE, METHOD FOR OPERATING ULTRASONIC OBSERVATION DEVICE, AND PROGRAM FOR OPERATING ULTRASONIC OBSERVATION DEVICE**

(57)  An ultrasound observation apparatus according to the present invention includes a frequency analysis unit, a feature calculation unit, and a feature image data generation unit. The frequency analysis unit calculates a plurality of frequency spectra by analyzing a frequency of a signal generated on the basis of an echo signal that is an electric signal converted from an ultrasound echo generated from ultrasound transmitted to an observation target and reflected from the observation target. The feature calculation unit calculates each of features of the plurality of frequency spectra, calculates corrected features of each of the frequency spectra by performing attenuation correction on the features of each of the frequency spectra, on each of a plurality of attenuation rate candidate values giving different attenuation characteristics when the ultrasound propagates through the observation target, and sets an optimum attenuation rate for the observation target from among the plurality of attenuation rate candidate values, using the corrected features. The feature image data generation unit generates feature image data for displaying the corrected features based on the optimum attenuation rate in association with visual information, together with an ultrasound image generated from the echo signal.

FIG.1

EP 3 167 809 A1

**Description**

Field

The present invention relates to an ultrasound observation apparatus for observing tissue as an observation target using ultrasound, a method for operating the ultrasound observation apparatus, and a program for operating the ultrasound observation apparatus.

Background

In order to observe characteristics of a living tissue or of a material, as an observation target, ultrasound is applied in some cases. Specifically, ultrasound is transmitted onto the observation target and reflected from the observation target, as an ultrasound echo. Information on characteristics of the observation target is obtained by performing predetermined signal processing on the reflected ultrasound echo.

Intensity of ultrasound attenuates when the ultrasound propagates through the observation target. There is a known technique of determining characteristics of a material as an observation target using attenuation (for example, refer to Patent Literature 1). This technique converts an electric signal corresponding to the ultrasound echo into an amplitude spectrum of frequency domain, and calculates an attenuation amount by comparing this amplitude spectrum with a predetermined reference amplitude spectrum, and determines the characteristics of a material by fitting the attenuation amount with an attenuation model that depends on the characteristics of the material.

Citation List

Patent Literature

Patent Literature 1: JP-T-2008-545123

Summary

Technical Problem

In the technique disclosed in Patent Literature 1, the reference amplitude spectrum is set using a reference object (reference piece) that has a same shape as that of the observation target and has ultrasound velocity equivalent to that of the observation target and that is formed of a material that causes substantially no attenuation of ultrasound. While the method of determining the characteristics of the observation target using the reference amplitude spectrum set in this manner would be effective for a material having a regular structure, it is difficult to apply this method to a living tissue having, by itself, an irregular structure.

In addition, when determining the characteristics of a material using the technique in the above-described Patent Literature 1, it is necessary to perform a large amount of calculation using a plurality of different attenuation models corresponding to factors of attenuation, such as scattering and absorption.

The present invention has been made in view of the foregoing, and an object of the invention is to provide an ultrasound observation apparatus, a method for operating the ultrasound observation apparatus, and a program for operating the ultrasound observation apparatus that are capable of obtaining, with simple calculation, attenuation characteristics of ultrasound that conforms to an observation target and capable of performing observation using the attenuation characteristics.

Solution to Problem

In order to solve the above-described problem and achieve the object, an ultrasound observation apparatus according to the invention includes: a frequency analysis unit configured to analyze a frequency of a signal generated based on an echo signal that is obtained by converting an ultrasound echo into an electric signal, the ultrasound echo having been generated from ultrasound transmitted to an observation target and reflected from the observation target, thereby to calculate a plurality of frequency spectra; a feature calculation unit configured to: calculate features of the plurality of frequency spectra; perform attenuation correction for eliminating an effect of ultrasound attenuation on the features of the frequency spectra, for each of a plurality of attenuation rate candidate values giving different attenuation characteristics when the ultrasound propagates through the observation target, thereby to calculate corrected features of the frequency spectra; and set an optimum attenuation rate for the observation target from among the plurality of attenuation rate candidate values, using the corrected features; and a feature image data generation unit configured to generate feature image data for displaying the corrected features based on the optimum attenuation rate in association with visual information, together with an ultrasound image generated from the echo signal.

In the ultrasound observation apparatus according to the above-described invention, the feature calculation unit is configured to: approximate each of the frequency spectra by an nth-order expression (n is a positive integer) to calculate the features; calculate statistical dispersion of the corrected features for each of the attenuation rate candidate values; and set an attenuation rate candidate value having minimum statistical dispersion as the optimum attenuation rate.

In the ultrasound observation apparatus according to the above-described invention, the feature calculation unit is configured to: approximate a predetermined frequency band of the frequency spectra by a linear expression; calculate, as the features, one or more of intercept of the linear expression, slope of the linear expression, and a mid-band fit, so as to include one of the slope and the mid-band fit, the mid-band fit being a value of the linear expression at an intermediate frequency of the frequency band; and set the optimum attenuation rate based on one of the slope and the mid-band fit.

In the ultrasound observation apparatus according to the above-described invention, the feature calculation unit is configured to: set the optimum attenuation rate based on the slope if the slope is calculated as the features; and set the optimum attenuation rate based on the mid-band fit if the mid-band fit is calculated as the features.

In the ultrasound observation apparatus according to the above-described invention, the feature calculation unit is configured to: calculate the statistical dispersion as a function of the attenuation rate candidate values; and set the attenuation rate candidate value having the minimum statistical dispersion in the function, as the optimum attenuation rate.

In the ultrasound observation apparatus according to the above-described invention, the feature calculation unit is configured to set the optimum attenuation rate for all frames of the ultrasound image.

In the ultrasound observation apparatus according to the above-described invention, the feature calculation unit is configured to: set the optimum attenuation rate every two or more predetermined number of frames of the ultrasound image; and calculate the features of the frequency spectra, in a frame for which the optimum attenuation rate is not set, using the optimum attenuation rate that is lastly set previous to the frame.

In the ultrasound observation apparatus according to the above-described invention, the feature calculation unit is configured to: calculate an optimum attenuation rate equivalent value corresponding to the optimum attenuation rate for all frames of the ultrasound image; and set the optimum attenuation rate based on the optimum attenuation rate equivalent value calculated for two or more predetermined number of frames.

In the ultrasound observation apparatus according to the above-described invention, the feature image data contains information on the optimum attenuation rate.

The ultrasound observation apparatus according to the above-described invention further includes a display unit configured to display a feature image corresponding to the feature image data.

The ultrasound observation apparatus according to the above-described invention further includes an input unit configured to receive input for setting a target region for which the frequency spectra are calculated by the frequency analysis unit. The frequency analysis unit is configured to calculate the frequency spectra based on the ultrasound echo reflected from the target region.

In the ultrasound observation apparatus according to the above-described invention, the feature calculation unit is configured to set the optimum attenuation rate using data with a dynamic range that is wider than a dynamic range of data used by the feature image data generation unit.

A method for operating an ultrasound observation apparatus according to the invention includes: a frequency analysis step of analyzing, by a frequency analysis unit, a frequency of a signal generated based on an echo signal that is obtained by converting an ultrasound echo into an electric signal, the ultrasound echo having been generated from ultrasound transmitted to an observation target and reflected from the observation target, thereby to calculate a plurality of frequency spectra; a feature calculation step of calculating, by a feature calculation unit, features of the plurality of frequency spectra, performing attenuation correction for eliminating an effect of ultrasound attenuation on the features of the frequency spectra, for each of a plurality of attenuation rate candidate values giving different attenuation characteristics when the ultrasound propagates through the observation target, thereby to calculate corrected features of the frequency spectra, and setting an optimum attenuation rate for the observation target from among the plurality of attenuation rate candidate values, using the corrected features; and a feature image data generation step of generating, by a feature image data generation unit, feature image data for displaying the corrected features based on the optimum attenuation rate in association with visual information, together with an ultrasound image generated from the echo signal.

A program for operating an ultrasound observation apparatus causes the ultrasound observation apparatus to execute: a frequency analysis step of analyzing, by a frequency analysis unit, a frequency of a signal generated based on an echo signal that is obtained by converting an ultrasound echo into an electric signal, the ultrasound echo having been generated from ultrasound transmitted to an observation target and reflected from the observation target, thereby to calculate a plurality of frequency spectra; a feature calculation step of calculating, by a feature calculation unit, features of the plurality of frequency spectra, performing attenuation correction for eliminating an effect of ultrasound attenuation

on the features of the frequency spectra, for each of a plurality of attenuation rate candidate values giving different attenuation characteristics when the ultrasound propagates through the observation target, thereby to calculate corrected features of the frequency spectra, and setting an optimum attenuation rate for the observation target from among the plurality of attenuation rate candidate values, using the corrected features; and a feature image data generation step of generating, by a feature image data generation unit, feature image data for displaying the corrected features based on the optimum attenuation rate in association with visual information, together with an ultrasound image generated from the echo signal. Advantageous Effects of Invention

According to the present invention, an optimum attenuation rate for an observation target is set from among a plurality of attenuation rate candidate values giving different attenuation characteristics when the ultrasound propagates through the observation target, and features of frequency spectra are calculated by performing attenuation correction using the optimum attenuation rate. Accordingly, it is possible to obtain, with simple calculation, attenuation characteristics of ultrasound that conforms to the observation target, and to perform observation using the attenuation characteristics.

Brief Description of Drawings

FIG. 1 is a block diagram illustrating a configuration of an ultrasound observation apparatus according to an embodiment of the present invention.
FIG. 2 is a graph illustrating a relationship between a reception depth and an amplification factor in amplification processing performed by a signal amplification unit of an ultrasound observation apparatus according to the embodiment of the present invention.
FIG. 3 is a graph illustrating a relationship between a reception depth and an amplification factor in amplification correction processing performed by an amplification correction unit of an ultrasound observation apparatus according to the embodiment of the present invention.
FIG. 4 is a schematic diagram illustrating data arrangement of a sound ray of an ultrasound signal.
FIG. 5 is a graph illustrating an exemplary frequency spectrum calculated by a frequency analysis unit of an ultrasound observation apparatus according to the embodiment of the present invention.
FIG. 6 is a graph illustrating a line having, as a parameter, corrected feature obtained by correction by an attenuation correction unit of an ultrasound observation apparatus according to the embodiment of the present invention.
FIG. 7 is a graph schematically illustrating exemplary distribution of corrected features after attenuation correction on the basis of two different attenuation rate candidate values, performed on a same observation target.
FIG. 8 is a flowchart illustrating an outline of processing performed by an ultrasound observation apparatus according to the embodiment of the present invention.
FIG. 9 is a flowchart illustrating an outline of processing executed by a frequency analysis unit of an ultrasound observation apparatus according to the embodiment of the present invention.
FIG. 10 is a graph illustrating an outline of processing performed by an optimum attenuation rate setting unit of an ultrasound observation apparatus according to the embodiment of the present invention.
FIG. 11 is a diagram schematically illustrating an exemplary display of a feature image on a display unit of an ultrasound observation apparatus according to the embodiment of the present invention.
FIG. 12 is a graph illustrating an outline of processing performed by an optimum attenuation rate setting unit of an ultrasound observation apparatus according to first modification of the embodiment of the present invention.

Description of Embodiments

Hereinafter, modes for carrying out the present invention (hereinafter, referred to as embodiment(s)) will be described with reference to the attached drawings.

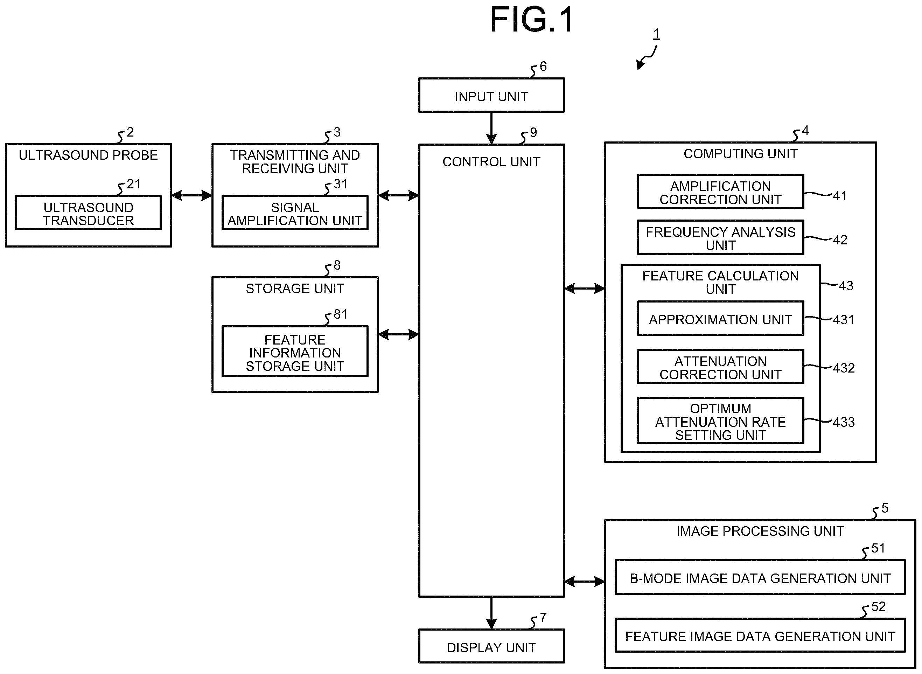

FIG. 1 is a block diagram illustrating a configuration of an ultrasound observation apparatus according to an embodiment of the present invention. An ultrasound observation apparatus 1 illustrated in the diagram is an apparatus for observing an observation target using ultrasound.

The ultrasound observation apparatus 1 includes an ultrasound probe 2, a transmitting and receiving unit 3, a computing unit 4, an image processing unit 5, an input unit 6, a display unit 7, a storage unit 8, and a control unit 9. The ultrasound probe 2 outputs an ultrasound pulse to an observation target and receives an ultrasound echo reflected from the observation target. The transmitting and receiving unit 3 performs transmission and reception of an electric signal with the ultrasound probe 2. The computing unit 4 performs predetermined calculation on an electrical echo signal that is an electric signal converted from the ultrasound echo. The image processing unit 5 generates image data corresponding to the electrical echo signal. The input unit 6 includes a user interface such as a keyboard, a mouse, and a touch panel, and receives input of various types of information. The display unit 7 includes a display panel formed of liquid crystal or organic electro luminescence (EL) and displays various types of information including an image generated by the image processing unit 5. The storage unit 8 stores various information needed for ultrasound observation. The control unit controls operation of the ultrasound observation apparatus 1.

The ultrasound observation apparatus 1 includes the ultrasound probe 2 and a processing apparatus (processor). The ultrasound probe 2 includes an ultrasound transducer 21. The ultrasound probe 2 is removably connected to the processing apparatus, on which the above-described portions other than the ultrasound probe 2 are provided. In a case where the observation target is a living tissue, the ultrasound probe 2 may take any form of an external probe configured to emit ultrasound from a surface of the living body, a miniature ultrasound probe including a long-shaft insertion section to be inserted into intraluminal portions such as the gastrointestinal tract, the biliopancreatic duct, and the blood vessel, and an ultrasound endoscope configured to further include an optical system in addition to an intraluminal ultrasound probe. In a case where the form of the ultrasound endoscope is taken from among these, the ultrasound transducer 21 is provided on a distal end side of the insertion section of the intraluminal ultrasound probe, which is removably connected to the processing apparatus on the proximal end side.

The ultrasound transducer 21 converts an electrical pulse signal received from the transmitting and receiving unit 3 into an ultrasound pulse (acoustic pulse), and also converts an ultrasound echo reflected from the external observation target, into an electrical echo signal. The ultrasound probe 2 may cause the ultrasound transducer 21 to perform mechanical scan, or may provide, as the ultrasound transducer 21, a plurality of elements in an array, and may cause the ultrasound transducer to perform electronic scan by electronically switching elements related to transmission/reception or imposing delay onto transmission/reception of each of elements. In some embodiments, one of different types of ultrasound probes 2 can be selected when in use.

The transmitting and receiving unit 3 is electrically connected with the ultrasound probe 2, transmits an electrical pulse signal to the ultrasound probe 2, and receives an echo signal as an electrical reception signal, from the ultrasound probe 2. Specifically, the transmitting and receiving unit 3 generates an electrical pulse signal on the basis of a preset waveform and transmission timing and transmits the generated pulse signal to the ultrasound probe 2.

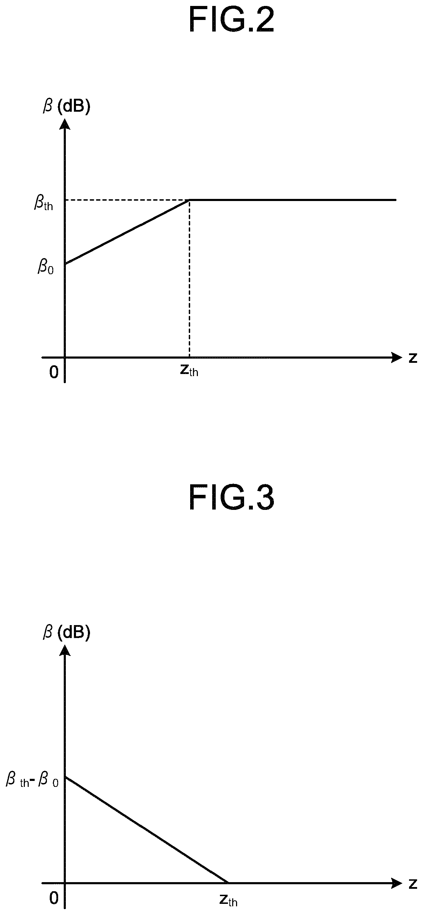

The transmitting and receiving unit 3 includes a signal amplification unit 31 that amplifies an echo signal. The signal amplification unit 31 performs sensitivity time control (STC) correction that amplifies an echo signal having a larger reception depth by using a higher amplification factor. FIG. 2 is a graph illustrating a relationship between a reception depth and an amplification factor in STC correction processing performed by the signal amplification unit 31. A reception depth z illustrated in FIG. 2 is an amount calculated on the basis of elapsed time from a point of starting reception of ultrasound. As illustrated in FIG. 2, in a case where the reception depth z is smaller than a threshold $z_{th}$, an amplification factor $\beta$ (dB) increases linearly from $\beta_0$ to $\beta_{th}$ ($> \beta_0$) along with an increase in the reception depth z. In a case where the reception depth z is the threshold $z_{th}$ or above, the amplification factor $\beta$ (dB) takes a fixed value $\beta_{th}$. The value of the threshold $z_{th}$ is a value at which an ultrasound signal received from the observation target has nearly completely attenuated and noise is dominant. More typically, in a case where the reception depth z is smaller than the threshold $z_{th}$, the amplification factor may preferably increase monotonically along with an increase in the reception depth z.

The transmitting and receiving unit 3 performs processing such as filtering on the echo signal amplified by the signal amplification unit 31, thereafter, generates digital high-frequency signal, namely, a radio frequency (RF) signal of time domain by performing A/D conversion on the signal, and outputs the generated signal. In a case where the ultrasound probe 2 is configured to perform scanning electronically with the ultrasound transducer 21 having a plurality of elements arranged in array, the transmitting and receiving unit 3 includes a beam-combining multi-channel circuit corresponding to the plurality of elements.

The computing unit 4 includes an amplification correction unit 41, a frequency analysis unit 42, and a feature calculation unit 43. The amplification correction unit 41 performs amplification correction on the digital RF signal generated by the transmitting and receiving unit 3 such that an amplification factor is fixed regardless of the reception depth. The frequency analysis unit 42 calculates a frequency spectrum by performing frequency analysis by applying fast Fourier transform (FFT) to the amplification-corrected digital RF signal. The feature calculation unit 43 calculates feature of the frequency spectrum. The computing unit 4 is formed with a central processing unit (CPU), various types of calculation circuits, or the like.

FIG. 3 is a graph illustrating a relationship between a reception depth and an amplification factor in amplification correction processing performed by the amplification correction unit 41. As illustrated in FIG. 3, the amplification factor $\beta$ (dB) in the amplification processing performed by the amplification correction unit 41 takes a maximum value $\beta_{th} - \beta_0$ when the reception depth z is zero, decreases linearly until the reception depth z reaches the threshold $z_{th}$ from zero, and takes zero when the reception depth z is not less than the threshold $z_{th}$. Since the amplification correction unit 41 performs the amplification correction on the digital RF signal using the amplification factor as defined above, it is possible to offset the effect of STC correction by the signal amplification unit 31 and to output a signal with a constant amplification factor $\beta_{th}$. The relationship between the reception depth z and the amplification factor $\beta$ performed by the amplification correction unit 41 understandably differs depending upon the relationship between the reception depth and the amplification factor in the signal amplification unit 31.

The reason for performing such amplification correction will be described. The STC correction is correction processing to eliminate the effect of attenuation from amplitude of an analog signal waveform by amplifying the amplitude of the analog signal waveform uniformly across an overall frequency band, with an amplification factor monotonically increasing with respect to the depth. Accordingly, in the case of generating a B-mode image in which echo signal amplitude is converted into luminance and displayed and in the case of scanning a uniform tissue, performing STC correction produces a fixed luminance value regardless of depth. That is, it is possible to eliminate the effect of attenuation, from the luminance value of the B-mode image.

In contrast, when utilizing the results of calculation and analysis of the frequency spectrum of the ultrasound as in the embodiment, it is difficult, even with the STC correction, to accurately eliminate the effect of attenuation caused by the propagation of the ultrasound. The reason is that the attenuation amount differs depending upon the frequency (refer to expression (1) described below) but the amplification factor of STC correction changes only for the distance, namely, does not depend upon the frequency.

As described above, when utilizing the results of calculation and analysis of the frequency spectrum of the ultrasound, it is difficult, even with the STC correction, to accurately eliminate the effect of attenuation caused by the propagation of the ultrasound. In order to address such a situation, one possibility may be that, while an STC-corrected reception signal is output when generating a B-mode image, a reception signal that has not been subjected to STC correction is output by performing new transmission different from the transmission to generate a B-mode image when generating an image based on the frequency spectrum. In this case, however, the frame rate of image data generated based on the reception signal may be decreased.

To cope with this, in the embodiment, the amplification correction unit 41 performs correction of an amplification factor on the STC-corrected signal for B-mode image, in order to eliminate the effect of STC correction, while maintaining the frame rate of the image data to be generated.

Amplification correction is performed on a digital RF signal based on an echo signal, and each sound ray (line data) of the amplification-corrected signal is sampled at predetermined time intervals to obtain an amplitude data group. The frequency analysis unit 42 performs fast Fourier transform on the amplitude data group to calculate the frequency spectra at a plurality of locations (data positions) on the sound ray.

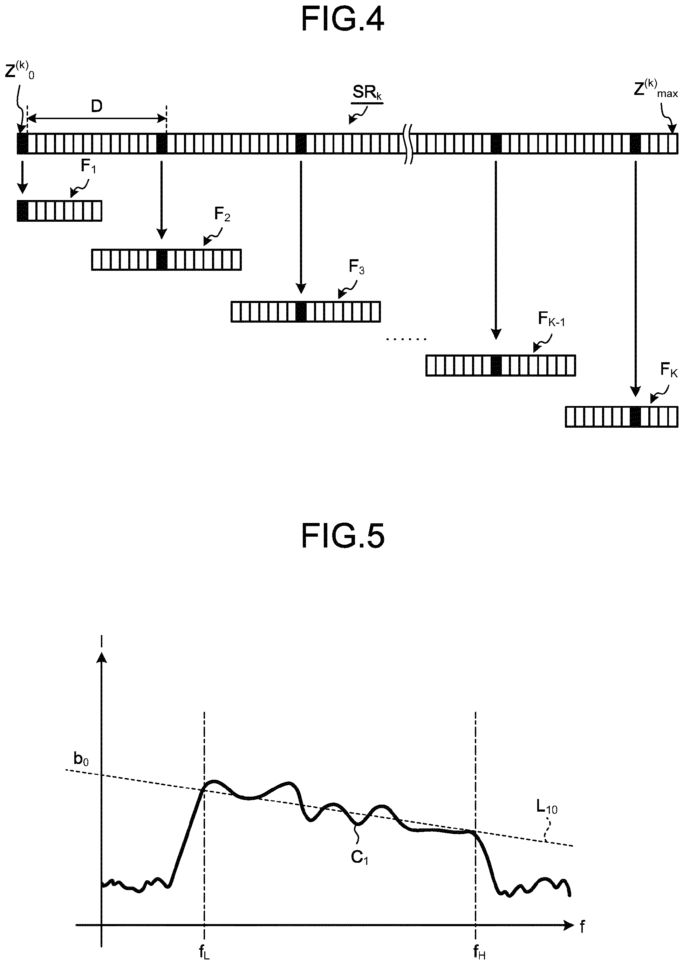

FIG. 4 is a schematic diagram illustrating data arrangement of a sound ray of an ultrasound signal. In a sound ray $SR_k$ illustrated in FIG. 4, a white or black rectangle indicates one set of data. The sound ray $SR_k$ is discretized with a time interval corresponding to a sampling frequency (e.g. 50 MHz) in A/D conversion performed by the transmitting and receiving unit 3. FIG. 4 illustrates a case where a first data position of the sound ray $SR_k$ with the number k is set as an initial value $Z^{(k)}_0$ in the reception depth z direction. It is however allowable to set the position of the initial value arbitrarily. A result of calculation by the frequency analysis unit 42 is obtained as a complex number and stored in the storage unit 8.

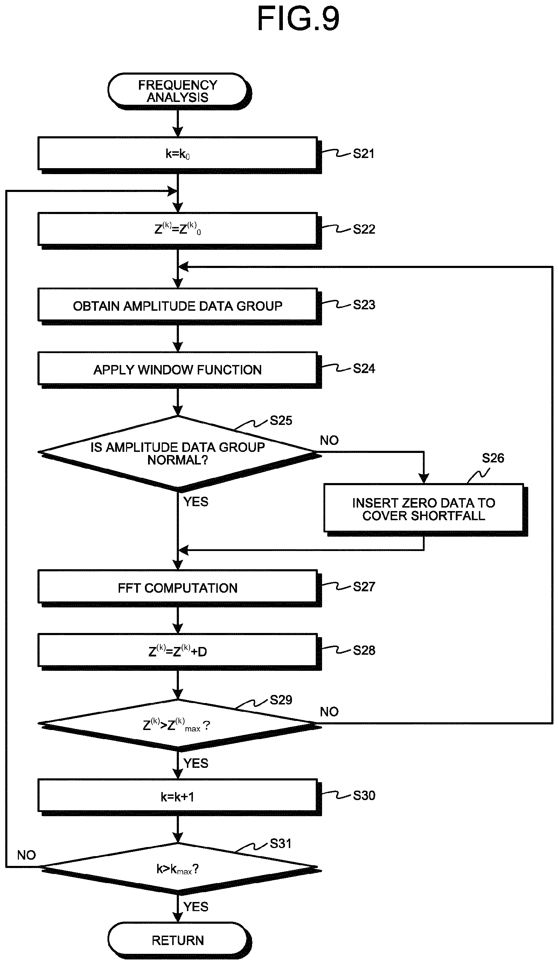

A data group $F_j$ (j = 1, 2, ···, K) illustrated in FIG. 4 is an amplitude data group as a target of fast Fourier transform. In general, in order to perform fast Fourier transform, it is necessary that the amplitude data group has the number of data that is power of two. In this sense, while the amplitude data group $F_j$ (j = 2, ···, K-1) has the number of data of 16 (= $2^4$), indicating it is a normal data group, the amplitude data groups $F_1$ and $F_K$ have the numbers of data of 9 and 12, respectively, indicating they are abnormal data groups. When fast Fourier transform is performed on an abnormal data group, processing of generating a normal amplitude data group is performed by inserting zero data by the extent of the shortage. This issue will be described in detail in the explanation of processing of the frequency analysis unit 42 (refer to FIG. 9).

FIG. 5 is a graph illustrating an exemplary frequency spectrum calculated by the frequency analysis unit 42. Herein, "frequency spectrum" means "frequency distribution of intensity at a specific reception depth z" obtained by performing fast Fourier transform (FFT computation) on the amplitude data group. In addition, "intensity" represents any of parameters such as echo signal voltage, echo signal power, ultrasound echo sound pressure, and ultrasound echo acoustic energy, amplitude or a time-integrated value of these parameters, or a combination of these.

In FIG. 5, the horizontal axis represents frequency f. In FIG. 5, the vertical axis represents common logarithm (decibel representation) $I = 10\log_{10}(I_0/I_c)$ of the amount obtained by dividing intensity $I_0$ by reference intensity $I_c$ (constant). In FIG. 5, the reception depth z is fixed. A line $L_{10}$ illustrated in FIG. 5 will be described below. Note that in the embodiment, each of curves and lines is formed of a set of discrete points.

On a frequency spectrum $C_1$ illustrated in FIG. 5, a lower limit frequency $f_L$ and an upper limit frequency $f_H$ of the frequency band to be used in the later calculation are parameters determined on the basis of the frequency band of the ultrasound transducer 21 and the frequency band of the pulse signal transmitted by the transmitting and receiving unit 3, exemplary frequency being $f_L$ = 3 MHz, $f_H$ = 10 MHz. Hereinafter, the frequency band determined, in FIG. 5, by the lower limit frequency $f_L$ and the upper limit frequency $f_H$ will be referred to as a "frequency band F".

In general, if the observation target is a living tissue, the frequency spectrum indicates different tendencies depending on the characteristics (attribute) of the living tissue after ultrasound scanning. This is because the frequency spectrum has a correlation with the size, number density, acoustic impedance, or the like, of a scatterer for scattering the ultrasound. Herein, exemplary "characteristics of the living tissue" includes malignant tumor (cancer), benign tumor, endocrine tumor, mucinous tumor, normal tissues, and vessels.

The feature calculation unit 43 calculates features of each of a plurality of frequency spectra, and performs attenuation correction for eliminating an effect of ultrasound attenuation, on the features of each of the frequency spectra (hereinafter, referred to as pre-correction feature) on each of a plurality of attenuation rate candidate values giving different attenuation characteristics when the ultrasound propagates through the observation target. With this attenuation correction, the feature calculation unit 43 calculates corrected features of each of the frequency spectra, and subsequently by using the corrected features, sets an optimum attenuation rate for the observation target from among the plurality of attenuation rate candidate values. The feature calculation unit 43 includes an approximation unit 431, an attenuation correction unit 432, and an optimum attenuation rate setting unit 433. The approximation unit 431 calculates pre-correction feature of a frequency spectrum by linearly approximating the frequency spectrum. The attenuation correction unit 432 calculates corrected feature by performing attenuation correction based on each of a plurality of attenuation rate candidate values, on pre-correction feature calculated by the approximation unit 431. The optimum attenuation rate setting unit 433 sets an optimum attenuation rate from among the plurality of attenuation rate candidate values on the basis of statistical dispersion of the corrected feature values calculated for all the frequency spectra by the attenuation correction unit 432.

The approximation unit 431 performs regression analysis of the frequency spectrum on a predetermined frequency band and approximates the frequency spectrum by a linear expression (regression line), thereby calculating pre-correction feature characterizing the linear expression used in approximation. For example, in the case of the frequency spectrum $C_1$ illustrated in FIG. 5, the approximation unit 431 performs regression analysis in the frequency band F, and approximates the frequency spectrum $C_1$ by a linear expression, thereby obtaining a regression line $L_{10}$. In other words, the approximation unit 431 calculates, as the pre-correction features, slope $a_0$ and intercept $b_0$ of the regression line $L_{10}$, and mid-band fit $c_0 = a_0 f_M + b_0$, namely, a central frequency value $f_M = (f_L + f_H)/2$ of the frequency band F, on the regression line.

Among the three pre-correction features, the slope $a_0$ has a correlation with the size of the ultrasound scatterer, and thus, is generally considered to have a smaller value as the scatterer size increases. The intercept $b_0$ has a correlation with the size of the scatterer, an acoustic impedance difference, number density (density) of the scatterer, or the like. Specifically, it is generally considered that the intercept $b_0$ has a larger value as the scatterer size increases, as the acoustic impedance increases, and as number density of the scatterer increases. The mid-band fit $c_0$ is an indirect parameter derived from the slope $a_0$ and the intercept $b_0$, and gives spectrum intensity on the center of an effective frequency band. Accordingly, the mid-band fit $c_0$ is considered to have a certain level of correlation with the scatterer size, an acoustic impedance difference, number density of the scatterer, and in addition to these, with luminance of a B-mode image. The feature calculation unit 43 may approximate the frequency spectrum by a second-order or higher order polynomial using regression analysis.

Correction performed by the attenuation correction unit 432 will be described. Typically, an attenuation amount A (f, z) of ultrasound is attenuation generated during ultrasound reciprocation between the reception depth 0 and the reception depth z, and defined as an intensity change (difference in decibel representation) between before and after reciprocation. The attenuation amount A (f, z) is empirically known to be proportional to a frequency in a uniform tissue, and is represented with the following expression (1).

$$A(f, z) = 2\alpha z f \quad \cdots \quad (1)$$

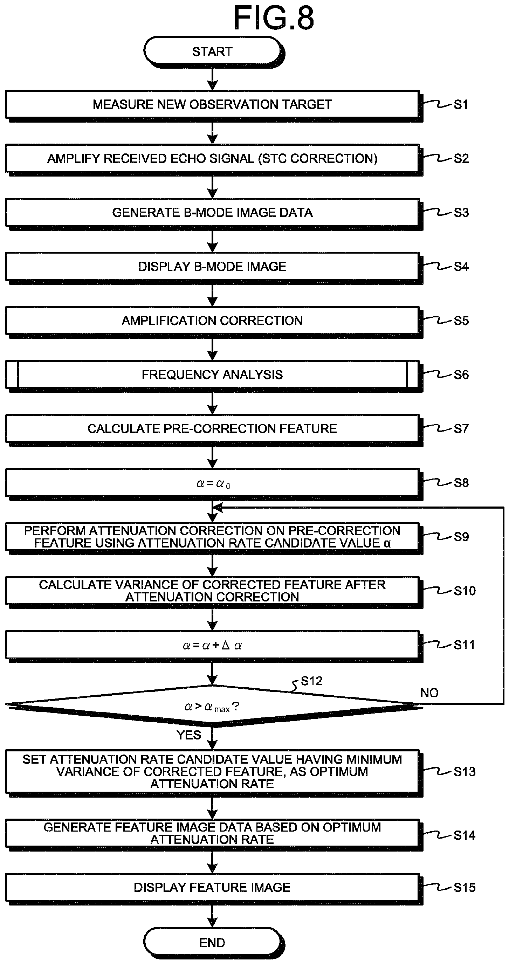

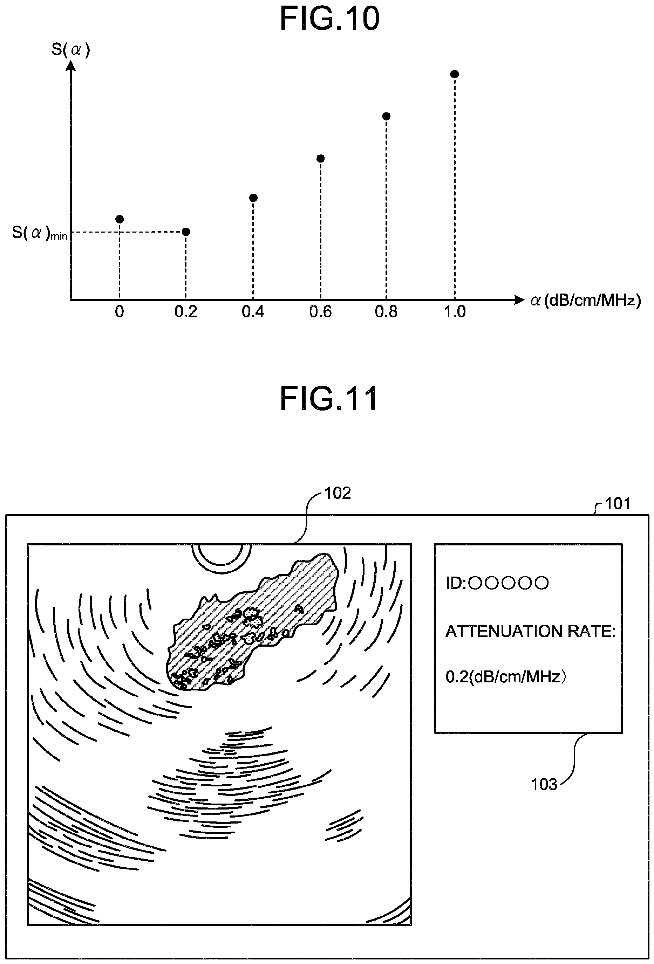

where, a proportionality constant $\alpha$ is an amount referred to as the attenuation rate. In addition, z represents the ultrasound reception depth, and f represents the frequency. In a case where the observation target is a living body, a specific value of the attenuation rate $\alpha$ is defined according to the sites on the living body. An exemplary unit of the attenuation rate $\alpha$ is dB/cm/MHz. In the embodiment, the attenuation correction unit 432 performs attenuation correction on each of a plurality of attenuation rate candidate values in order to set an attenuation rate (optimum attenuation rate) that is most suitable to the observation target. Details of the plurality of attenuation rate candidate values will be described below with reference to FIGS. 8 and 10.

The attenuation correction unit 432 calculates corrected features a, b, and c by performing attenuation correction according to expressions (2) to (4) below, on the pre-correction features (the slope $a_0$, intercept $b_0$, and the mid-band fit $c_0$) extracted by the approximation unit 431.

$$a = a_0 + 2\alpha z \quad \cdots \quad (2)$$

$$b = b_0 \quad \cdots \quad (3)$$

$$c = c_0 + A(f_M, z) = c_0 + 2\alpha z f_M \ (= a f_M + b) \quad \cdots \quad (4)$$

As clearly known from expressions (2) and (4), the attenuation correction unit 432 performs correction such that the correction amount increases as the ultrasound reception depth z increases. In addition, according to expression (3), correction regarding the intercept is identity transform. This is because the intercept is a frequency component corresponding to frequency 0 (Hz), so as not to be affected by attenuation.

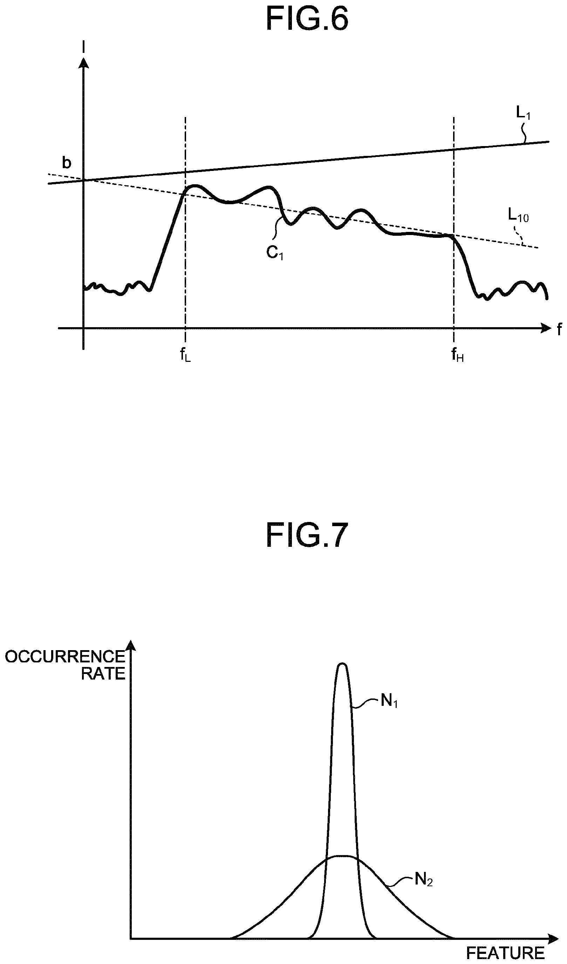

FIG. 6 is a graph illustrating a line having, as parameters, corrected features a, b, and c, obtained by the attenuation correction unit 432. A line $L_1$ is represented by

$$I = a f + b = (a_0 + 2\alpha z) \ f + b_0 \quad \cdots \quad (5).$$

As is clear from expression (5), the line $L_1$ has larger slope ($a > a_0$), and equal intercept ($b = b_0$) as compared with the line $L_{10}$ before attenuation correction.

The optimum attenuation rate setting unit 433 sets, as an optimum attenuation rate, the attenuation rate candidate value having minimum statistical dispersion of the corrected features calculated by the attenuation correction unit 432 for the all frequency spectra, for each of the attenuation rate candidate values. In the embodiment, variance is applied as the amount indicating the statistical dispersion. In this case, the optimum attenuation rate setting unit 433 sets the optimum attenuation rate candidate value having minimum variance as an optimum attenuation rate. Two of the three corrected features a, b, and c are independent from one another. In addition, the corrected feature b does not depend upon the attenuation rate. Therefore, in a case where an optimum attenuation rate is set for the corrected features a and c, the optimum attenuation rate setting unit 433 is only required to calculate variance of one of the corrected features a and c.

In this, however, it would be preferable that the corrected features to be used at an occasion where the optimum attenuation rate setting unit 433 sets the optimum attenuation rate are of the same type as the corrected features to be used at an occasion where a feature image data generation unit 52 generates feature image data. It would be more preferable to apply variance of corrected feature a in a case where the feature image data generation unit 52 generates feature image data using slope as corrected feature, and to apply variance of corrected feature c in a case where the feature image data generation unit 52 generates feature image data using mid-band fit as corrected feature. This is because the expression (1) that gives the attenuation amount A (f, z) is an expression that is unrealistic, and it would be more appropriate to use the following expression (6) in practice.

$$A(f, z) = 2\alpha z f + 2\alpha_1 z \quad \cdots \quad (6)$$

In the second term on the right-hand side in expression (6), $\alpha_1$ represents a coefficient indicating the magnitude of signal intensity change in proportion to the reception depth z of ultrasound, namely, a coefficient indicating signal intensity change generated due to nonuniformity of observation target tissue or channel number change at beam combining. Because of the existence of the second term on the right-hand side in expression (6), it would be possible to accurately correct attenuation with application of variance of the corrected feature value c in a case where a feature image is to be generated using the corrected feature c (refer to expression (4)). In contrast, in a case where the feature image is to be generated using the corrected feature a, namely, a coefficient that is proportional to the frequency f, it would be possible to correct attenuation accurately by excluding the effects of the second term on the right-hand side with application of variance of the corrected feature a. For example, in a case where unit of the attenuation rate $\alpha$ is dB/cm/MHz, the unit of a coefficient $\alpha_1$ is dB/cm.

The reason why it is possible to set an optimum attenuation rate on the basis of statistical dispersion will be described. In a case where an optimum attenuation rate for an observation target is applied, the features are presumably converged to a value inherent to the observation target, leading to small statistical dispersion, regardless of the distance between the observation target and the ultrasound transducer 21. In contrast, in a case where an attenuation rate candidate value that does not conform to the observation target is applied as an optimum attenuation rate, attenuation correction might become excessive or insufficient, and thus, would presumably cause deviation in the features according to the distance with the ultrasound transducer 21, and increase statistical dispersion of the features. Accordingly, it is reasonable to assume that the attenuation rate candidate value having the smallest statistical dispersion would be the optimum attenuation rate for the observation target.

FIG. 7 is a graph schematically illustrating exemplary distribution of corrected features after attenuation correction on the basis of two different attenuation rate candidate values, performed on a same observation target. In FIG. 7, the horizontal axis represents the corrected features and the vertical axis represents an occurrence rate. Two distribution curves $N_1$ and $N_2$ illustrated in FIG. 7 share the same sum of occurrence rate. In the case illustrated in FIG. 7, the distribution curve $N_1$ has smaller statistical dispersion of the features (smaller variance) and a steeper peak shape, as compared with the distribution curve $N_2$. Accordingly, in a case where an optimum attenuation rate is set from among the two attenuation rate candidate values corresponding to these two distribution curves $N_1$ and $N_2$, the optimum attenuation rate setting unit 433 sets the attenuation rate candidate value corresponding to the distribution curve $N_1$ as the optimum attenuation rate.

The image processing unit 5 includes a B-mode image data generation unit 51 and a feature image data generation unit 52. The B-mode image data generation unit 51 generates B-mode image data, namely, an ultrasound image displayed after being converted from amplitude of an echo signal into luminance. The feature image data generation unit 52 generates feature image data for displaying the feature based on an optimum attenuation rate set by the optimum attenuation rate setting unit 433, together with the B-mode image, in association with visual information.

The B-mode image data generation unit 51 performs, on digital signals, signal processing using known techniques including a band-pass filter, logarithmic transform, gain processing, and contrast processing, and generates B-mode image data by performing data decimation depending on a data step width defined in accordance with the display range of the image on the display unit 7 or by other methods. The B-mode image is a gray-scale image in which values of R (red), G (green) and B (blue), namely, variables when the RGB color system is employed as a color space, match with each other.

The feature image data generation unit 52 generates feature image data by superposing visual information regarding the features calculated by the feature calculation unit 43, onto each pixel of an image on the B-mode image data. The feature image data generation unit 52 allocates, for example, visual information corresponding to feature of the frequency spectrum calculated from an amplitude data group $F_j$, onto an image region corresponding to the data amount of one amplitude data group $F_j$ ($j = 1, 2, \cdots, K$) illustrated in FIG. 4.

The feature image data generation unit 52 generates a feature image by associating, for example, hue as visual information with any one of the above-described slope, intercept, and the mid-band fit. Alternatively, it is allowable to configure such that the feature image data generation unit 52 generates feature image data by associating hue with one of the two features selected from slope, intercept and the mid-band fit, and by associating contrast with the other. Visual information regarding the feature includes variables of a color space forming a predetermined color system such as hue, saturation, brightness, luminance value, and R, G, and B (representing red, green, and blue, respectively).

The storage unit 8 includes a feature information storage unit 81 configured to store a plurality of features calculated for each of frequency spectra by the attenuation correction unit 432 corresponding to the attenuation rate candidate values, and variance giving statistical dispersion of the plurality of features, in association with the attenuation rate candidate values.

In addition to the above-described information, the storage unit 8 stores, for example, information needed for amplification processing (relationship between amplification factor and reception depth, illustrated in FIG. 2), information needed for amplification correction processing (relationship between amplification factor and reception depth, illustrated in FIG. 3), information needed for attenuation correction processing (refer to expression (1)), and information on window function (Hamming, Hanning, Blackman, or the like) needed for frequency analysis processing.

The storage unit 8 also stores various types of programs including an operation program for executing a method for operating the ultrasound observation apparatus 1. The operation programs can be recorded in a computer-readable recording medium such as a hard disk, flash memory, CD-ROM, DVD-ROM, flexible disk, or the like, and can be distributed broadly. It is also possible to obtain the above-described various programs by downloading them via a communication network. Herein, the communication network refers to one implemented by, for example, a known public network, a local area network (LAN), a wide area network (WAN), regardless of wired or wireless.

The storage unit 8 with the above-described configuration is implemented using read only memory (ROM) in which various types of programs are pre-installed, random access memory (RAM) storing calculation parameters and data for each of processing, or the like.

The control unit 9 includes a central processing unit (CPU) having calculation and control functions, various calculation circuits, or the like. The control unit 9 reads, from the storage unit 8, information stored in the storage unit 8, and executes various types of calculation processing related to the method for operating the ultrasound observation apparatus 1, thereby performing overall control of the ultrasound observation apparatus 1. The control unit 9 and the computing unit 4 may share the common CPU or the like.

FIG. 8 is a flowchart illustrating outline of processing executed by the ultrasound observation apparatus 1 having the above-described configuration. The ultrasound observation apparatus 1 initially measures a new observation target by the ultrasound probe 2 (step S1). Specifically, the ultrasound transducer 21 of the ultrasound probe 2 converts an electrical pulse signal into an ultrasound pulse and sequentially transmits it to the observation target. Each of the ultrasound pulses is reflected from the observation target to generate an ultrasound echo. The ultrasound transducer 21 converts the ultrasound echo into an electrical echo signal. The frequency band of the pulse signal at this time is preferably a broadband substantially covering a linear response frequency band for electroacoustic conversion from pulse signals to ultrasound pulses on the ultrasound transducer 21. With this configuration, it is possible to perform accurate approximation in approximation processing of a frequency spectrum described below.

After receiving the echo signal from the ultrasound probe 2, the signal amplification unit 31 amplifies the echo signal (step S2). The signal amplification unit 31 performs, for example, echo signal amplification (STC correction) on the basis of the relationship between the amplification factor and the reception depth illustrated in FIG. 2. At this time, a frequency band for various types of processing of echo signal on the signal amplification unit 31 is preferably a broad band that substantially covers a linear response frequency band for acoustic-electric conversion from an ultrasound echo to an echo signal by the ultrasound transducer 21. A purpose of this is to enable accurate approximation in approximation processing of frequency spectrum described below.

Subsequently, the B-mode image data generation unit 51 generates B-mode image data using the echo signal amplified by the signal amplification unit 31 (step S3). Thereafter, the control unit 9 displays, on the display unit 7, the B-mode image corresponding to the generated B-mode image data (step S4).

The amplification correction unit 41 performs amplification correction on the signal output from the transmitting and receiving unit 3 such that the amplification factor is fixed regardless of the reception depth (step S5). For example, the amplification correction unit 41 performs amplification correction on the basis of a relationship between the amplification factor and the reception depth, illustrated in FIG. 3.

Thereafter, the frequency analysis unit 42 calculates the frequency spectrum for all the amplitude data groups by performing frequency analysis with FFT computation (step S6). FIG. 9 is a flowchart illustrating an outline of processing executed by the frequency analysis unit 42 in step S6. Hereinafter, frequency analysis processing will be described with reference to the flowchart in FIG. 9.

First, the frequency analysis unit 42 sets a counter k for identifying a sound ray as an analysis target, to $k_0$ (step S21).

Subsequently, the frequency analysis unit 42 sets (step S22) an initial value $Z^{(k)}_0$ of a data position (corresponding to reception depth) $Z^{(k)}$, representing a series of data group (amplitude data group) obtained for FFT computation. For example, FIG. 4 illustrates a case, as described above, where the first data position of the sound ray $SR_k$ has been set as the initial value $Z^{(k)}_0$.

Thereafter, the frequency analysis unit 42 obtains an amplitude data group to which the data position $Z^{(k)}$ belongs (step S23), and applies a window function stored by the storage unit 8 to the obtained amplitude data group (step S24). By applying the window function to the amplitude data group in this manner, it is possible to avoid discontinuity of the amplitude data group on a border and prevent occurrence of artifacts.

Subsequently, the frequency analysis unit 42 determines whether the amplitude data group of the data position $Z^{(k)}$ is a normal data group (step S25). As discussed with reference to FIG. 4, it is necessary that the amplitude data group has the number of data that is power of two. Hereinafter, the number of data of the normal amplitude data group is determined to be $2^n$ (n: positive integer). Setting in the embodiment is performed such that the data position $Z^{(k)}$ may be arranged at a center of the amplitude data group to which $Z^{(k)}$ belongs, as much as possible. Specifically, since the number of data of the amplitude data group is $2^n$, $Z^{(k)}$ is set to a $2^n/2 (= 2^{n-1})$ th position close to the center of the amplitude data group. In this case, the amplitude data group being normal means that data having the number of $2^{n-1} -1 (= N)$ exist on a more front side than the data position $Z^{(k)}$, and that the data having the number of $2^{n-1} (= M)$ exist on a more rear side than the data position $Z^{(k)}$. In the case illustrated in FIG. 4, the amplitude data groups $F_2$, and $F_3$ are both normal. Note that FIG. 4 exemplifies a case of n = 4 (N = 7 and M = 8).

In a case where the result of determination in step S25 indicates that the amplitude data group of the data position $Z^{(k)}$ is normal (step S25: Yes), the frequency analysis unit 42 proceeds to step S27 described below.

In a case where the result of determination in step S25 indicates that the amplitude data group of the data position $Z^{(k)}$ is not normal (step S25: No), the frequency analysis unit 42 generates a normal amplitude data group (step S26) by inserting zero data to cover the shortfall. The window function has been applied to the amplitude data group determined to be not normal in step S25 (e.g. amplitude data group $F_1$ and $F_K$ in FIG. 4) before addition of the zero data. Therefore, insertion of zero data to the amplitude data group would not cause discontinuity of data. After step S26, the frequency analysis unit 42 proceeds to step S27 to be described below.

In step S27, the frequency analysis unit 42 obtains a frequency spectrum as frequency distribution of amplitude by performing FFT computation using the amplitude data group (step S27). The frequency spectrum $C_1$ illustrated in FIG. 5 is exemplary frequency spectrum obtained as a result of step S27.

Subsequently, the frequency analysis unit 42 changes the data position $Z^{(k)}$ by a step width D (step S28). The step width D is assumed to be pre-stored in the storage unit 8. FIG. 4 illustrates an exemplary case of D = 15. The step width D is desirably equal to the data step width used in generation of B-mode image data by the B-mode image data generation unit 51. However, when reduction of calculation on the frequency analysis unit 42 is desired, a greater value than the data step width may be set as the step width D.

Thereafter, the frequency analysis unit 42 determines (step S29) whether the data position $Z^{(k)}$ is greater than a maximum value $Z^{(K)}_{max}$ in the sound ray $SR_k$.

In a case where the data position $Z^{(k)}$ is greater than the maximum value $Z^{(k)}_{max}$ (step S29: Yes), the frequency analysis unit 42 increments the counter k by one (step S30). This means transition of processing to an adjacent sound ray.

In contrast, in a case where the data position $Z^{(k)}$ is the maximum value $Z^{(k)}_{max}$ or below (step S29: No), the frequency analysis unit 42 returns to step S23. In this manner, the frequency analysis unit 42 performs FFT computation for the sound ray $SR_k$, on $[(Z^{(k)}_{max} - Z^{(K)}_0 + 1)/D + 1]$ amplitude data groups. Herein, [X] represents a largest integer that does not exceed X.

After step S30, the frequency analysis unit 42 determines whether the counter k is greater than the maximum value $k_{max}$ (step S31). If the counter k is greater than $k_{max}$ (step S31: Yes), the frequency analysis unit 42 finishes a series of FFT processing. In contrast, the counter k is $k_{max}$ or below (step S31: No), the frequency analysis unit 42 returns to step S22.

In this manner, the frequency analysis unit 42 performs a plurality of times of FFT computations for each of sound rays having the number ($k_{max} - k_0 + 1$) within a analysis target region.

In the above description, the frequency analysis unit 42 performs frequency analysis processing on all regions that have received an ultrasound signal. Alternatively, however, it is also possible to configure such that the input unit 6 can accept input of setting for a region of interest divided by a predetermined depth size and sound ray size, and that frequency analysis processing is performed within the set region of interest alone.

After the above-described frequency analysis processing in step S6, the feature calculation unit 43 calculates pre-correction features of each of the plurality of frequency spectra, and performs attenuation correction for eliminating an effect of ultrasound attenuation, on the pre-correction features of each of the frequency spectra for each of a plurality of attenuation rate candidate values giving different attenuation characteristics when the ultrasound propagates through the observation target, thereby calculating corrected features of each of the frequency spectra. The feature calculation unit 43, subsequently, sets an optimum attenuation rate for the observation target from among the plurality of attenuation rate candidate values using the corrected features (steps S7 to S13). Herein, processing in step S7 to S13 will be described in detail.

In step S7, the approximation unit 431 performs regression analysis on each of the plurality of frequency spectra calculated by the frequency analysis unit 42, thereby calculating pre-correction features corresponding to each of the frequency spectra (step S7). Specifically, the approximation unit 431 performs regression analysis on each of the frequency spectra to obtain approximation by a linear expression, thereby calculating, as pre-correction features, the slope $a_0$, the intercept $b_0$, and the mid-band fit $c_0$. For example, the line $L_{10}$ illustrated in FIG. 5 is a regression line obtained by the approximation unit 431 by performing approximation using regression analysis on the frequency spectrum $C_1$ of the frequency band F.

Thereafter, the optimum attenuation rate setting unit 433 sets an attenuation rate candidate value $\alpha$ to be applied to the attenuation correction described below, to a predetermined initial value $\alpha_0$ (step S8). This initial value $\alpha_0$ may be pre-stored in the storage unit 8 and the optimum attenuation rate setting unit 433 may refer to the storage unit 8.

Subsequently, using the attenuation rate candidate value $\alpha$, the attenuation correction unit 432 performs attenuation correction on the pre-correction features approximated for each of the frequency spectra by the approximation unit 431, to calculate corrected features. The attenuation correction unit 432 stores the corrected features together with the attenuation rate candidate value $\alpha$, into a feature information storage unit 81 (step S9). The line $L_1$ illustrated in FIG. 6 is an exemplary line obtained by attenuation correction processing performed by the attenuation correction unit 432

In step S9, the attenuation correction unit 432 performs calculation by substituting data position $Z = (f_{sp}/2v_s)$ Dn obtained by using data arrangement of the sound ray of the ultrasound signal, into the reception depth z in the above-described expressions (2) and (4). Herein, $f_{sp}$ represents a data sampling frequency, $v_s$ represents a sound velocity, D represents a data step width, n represents the number of data step from the first data of the sound ray till the data position of the amplitude data group as a processing target. For example, when the data sampling frequency $f_{sp}$ is 50 MHz, and the sound velocity $v_s$ is 1530 m/sec, and the step width D is 15 by employing data arrangement illustrated in FIG. 4, the result would be z = 0.2295n (mm).

The optimum attenuation rate setting unit 433 calculates variance of representative corrected feature among a plurality of corrected features obtained by attenuation correction performed on each of the frequency spectra by the attenuation correction unit 432, and stores the calculation result in association with the attenuation rate candidate value $\alpha$, into the feature information storage unit 81 (step S10). In a case where the corrected features are slope a, or mid-band fit c, the optimum attenuation rate setting unit 433 calculates, as described above, variance of any one of the corrected features a and c. In step S10, it would be preferable that, variance of corrected feature a is applied in a case where the feature image data generation unit 52 generates feature image data using slope as corrected feature, and it would be preferable that variance of corrected feature c is applied in a case where the feature image data generation unit 52 generates feature image data using mid-band fit as corrected feature.

Thereafter, the optimum attenuation rate setting unit 433 increases the attenuation rate candidate value $\alpha$ by $\Delta\alpha$ (step S11) and compares the attenuation rate candidate value $\alpha$ after increase with a predetermined maximum value $\alpha_{max}$ (step S12). As a result of comparison in step S12, in a case where the attenuation rate candidate value $\alpha$ is larger than the maximum value $\alpha_{max}$ (step S12: Yes), the ultrasound observation apparatus 1 proceeds to step S13. In contrast, as a result of comparison in step S12, in a case where the attenuation rate candidate value $\alpha$ is the maximum value $\alpha_{max}$ or below (step S12: No), the ultrasound observation apparatus 1 returns to step S9.

In step S13, the optimum attenuation rate setting unit 433 refers to variance for each of the attenuation rate candidate values stored in the feature information storage unit 81 and sets the attenuation rate candidate value with the minimum variance, as the optimum attenuation rate (step S13).

FIG. 10 is a graph illustrating an outline of processing performed by the optimum attenuation rate setting unit 433. This diagrams illustrates an exemplary relationship between the attenuation rate candidate value $\alpha$ and the variance $S(\alpha)$ where $\alpha_0 = 0$ (dB/cm/MHz), $\alpha_{max} = 1.0$ (dB/cm/MHz), and $\Delta\alpha = 0.2$ (dB/cm/MHz). In a case illustrated in FIG. 10, the variance takes its minimum value $S(\alpha)_{min}$ when the attenuation rate candidate value $\alpha$ is 0.2 (dB/cm/MHz). Accordingly, in the case illustrated in FIG. 10, the optimum attenuation rate setting unit 433 sets $\alpha = 0.2$ (dB/cm/MHz) as the optimum attenuation rate.

The feature image data generation unit 52 generates feature image data (step S14) by superposing visual information (for example, hue) associated with the corrected features based on the optimum attenuation rate set in step S13 and by adding optimum attenuation rate information, onto each of the pixels of the B-mode image data generated by the B-mode image data generation unit 51.

Thereafter, under the control of the control unit 9, the display unit 7 displays a feature image corresponding to the feature image data generated by the feature image data generation unit 52 (step S15). FIG. 11 is a diagram schematically illustrating an exemplary display of the feature image on the display unit 7. A feature image 101 illustrated in FIG. 11 includes a superposed image display unit 102 and an information display unit 103. The superposed image display unit 102 displays an image generated by superposing visual information related to the feature, onto a B-mode image B. The information display unit 103 displays identification information of the observation target and information on the attenuation rate candidate value that has been set as an optimum attenuation rate. It is allowable to display also information of feature, information on approximation expression, image information such as gain and contrast, on the information display unit 103. Furthermore, it is allowable to display a B-mode image corresponding to the feature image, together with the feature image, side by side. It is also allowable to configure such that the input unit 6 can receive an instruction signal indicating whether the attenuation rate candidate value information is displayed.

In a series of processing described above (steps S1 to S15), it is allowable to configure so as to execute processing in step S4 and processing in steps S5 to S13 in parallel with each other.

According to the embodiment of the present invention described above, an optimum attenuation rate for an observation target is set from among a plurality of attenuation rate candidate values giving different attenuation characteristics when the ultrasound propagates through the observation target, and features of each of a plurality of frequency spectra are calculated by performing attenuation correction using the optimum attenuation rate. Accordingly, it is possible to obtain attenuation characteristics of ultrasound that conforms to the observation target, with simple calculation, and to perform observation using the attenuation characteristics.

Furthermore, according to the embodiment, the optimum attenuation rate is set on a basis of statistical dispersion of corrected features obtained by attenuation correction performed on each of the frequency spectra. Accordingly, it is possible to reduce the amount of computation as compared with a conventional technique in which fitting is performed with a plurality of attenuation models.

Furthermore, according to the embodiment, it is possible to set an optimum attenuation rate even when the attenuation rate conforming to the observation target is unknown.

(First Modification of Embodiment)

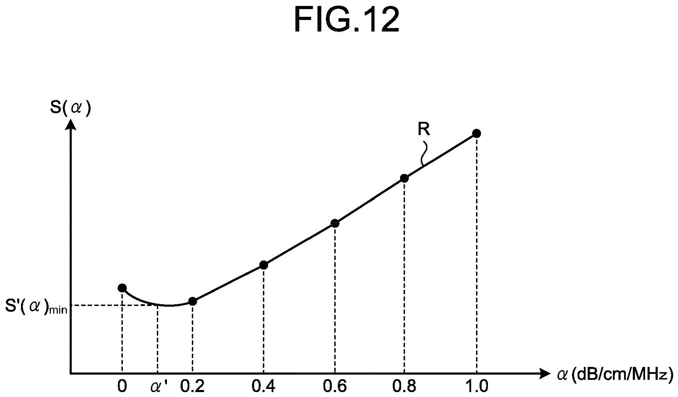

FIG. 12 is a graph illustrating an outline of processing performed by an optimum attenuation rate setting unit of the ultrasound observation apparatus according to a first modification of the embodiment. FIG. 12 illustrates an exemplary relationship between the attenuation rate candidate value $\alpha$ and variance $S(\alpha)$ where $\alpha_0 = 0$ (dB/cm/MHz), $\alpha_{max} = 1.0$ (dB/cm/MHz), and $\Delta\alpha = 0.2$ (dB/cm/MHz). In this example, the values of the variance $S(\alpha)$ when the attenuation rate candidate values $\alpha = 0, 0.2, 0.4, 0.6, 0.8,$ and 1.0 (dB/cm/MHz) are equal to the values in FIG. 10, respectively. In the first modification, the approximation unit 431 performs regression analysis before the optimum attenuation rate setting unit 433 sets an optimum attenuation rate, thereby calculating a curve R that interpolates the variance $S(\alpha)$ on the attenuation rate candidate value $\alpha$. Thereafter, the optimum attenuation rate setting unit 433 calculates a minimum value $S(\alpha)'_{min}$ for the curve R, in a range of 0 (dB/cm/MHz) $\leq \alpha \leq$ 1.0 (dB/cm/MHz), and sets the attenuation rate candidate value $\alpha'$ at this time, as an optimum attenuation rate. In the case illustrated in FIG. 12, the optimum attenuation rate $\alpha'$

is a value between 0 (dB/cm/MHz) and 0.2 (dB/cm/MHz).

(Second Modification of Embodiment)

Next, a second modification of the embodiment of the present invention will be described. In the present modification example, the optimum attenuation rate setting unit 433 sets an optimum attenuation rate in a dynamic range broader than the dynamic range at the time when the optimum attenuation rate setting unit 433 displays as the feature image.

Specifically, when the display dynamic range of the image generated by the feature image data generation unit 52 is 70 dB, the feature calculation unit 43 performs attenuation calculation processing in a dynamic range (for example, 100 dB) greater than this dynamic range (70 dB). For example, while the feature image data generation unit 52 uses an 8-bit fixed point system, the feature calculation unit 43 uses a 32-bit floating point system to perform attenuation calculation processing from calculation of the feature to setting of the optimum attenuation rate.

According to the second modification, it is possible to enhance calculation accuracy as compared with the attenuation calculation processing using the fixed point system. By performing processing from calculation of the pre-correction feature to the generation of a quadratic curve based on variance, with higher accuracy, it is possible to calculate the optimum attenuation rate with higher accuracy.

Embodiments of the present invention have been described hereinabove, however, the present invention is not intended to be limited to the above-described embodiments. For example, the optimum attenuation rate setting unit 433 may calculate equivalent values of the optimum attenuation rates equivalent value corresponding to the optimum attenuation rates for all frames of an ultrasound image, and may set, as an optimum attenuation rate, an average value, a center value, or a most frequent value of a predetermined number of optimum attenuation rate equivalent values including the optimum attenuation rate equivalent value in the latest frame. In this case, it is possible to stabilize the value with smaller change in the optimum attenuation rate, as compared with a case where the optimum attenuation rate is set for each of the frames.

The optimum attenuation rate setting unit 433 may set an optimum attenuation rate at predetermined frame intervals of an ultrasound image. With this configuration, it is possible to significantly reduce the amount of computation. In this case, it is possible to use an optimum attenuation rate value last set until the next optimum attenuation value can be set.

Moreover, the target region for which statistical dispersion is calculated may be set for each of sound rays or a region having a predetermined reception depth or above. It is allowable to configure such that setting of these regions can be received by the input unit 6.

Alternatively, the optimum attenuation rate setting unit 433 may separately set an optimum attenuation rate for each of inside of the set region of interest and outside of the region of interest.

The input unit 6 may receive input of setting change of the initial value $\alpha_0$ of the attenuation rate candidate value.

As an amount that provides statistical dispersion, it would also possible to apply any of a difference of maximum-minimum values of the features in standard deviation or population, and a half width of distribution of the features. As an amount that provides statistical dispersion, application of the inverse of the variance would be assumed. In this case, it is understandable that the attenuation rate candidate value in which the value takes the maximum value would be the optimum attenuation rate.

Alternatively, it would be possible that the optimum attenuation rate setting unit 433 calculates statistical dispersion for each of the plurality of types of corrected features, and sets the attenuation rate candidate value at the time when the statistical dispersion is minimum, as the optimum attenuation rate.

Alternatively, the attenuation correction unit 432 may perform attenuation correction on the frequency spectrum using a plurality of attenuation rate candidate values, and thereafter the approximation unit 431 may perform regression analysis on each of the attenuation-corrected frequency spectra, thereby calculating the corrected features.

In this manner, the present invention may include various forms of embodiments without deviating from the technical spirit and scope of the general inventive concept as defined in the appended claims of this invention.

Industrial Applicability

As described above, an ultrasound observation apparatus, a method for operating the ultrasound observation apparatus, and a program for operating the ultrasound observation apparatus of the present invention are useful in obtaining, with simple calculation, attenuation characteristics of ultrasound that conforms to an observation target, and in performing observation using the attenuation characteristics.

Reference Signs List

| | |
|---|---|
| 1 | ULTRASOUND OBSERVATION APPARATUS |
| 2 | ULTRASOUND PROBE |
| 3 | TRANSMITTING AND RECEIVING UNIT |
| 4 | COMPUTING UNIT |
| 5 | IMAGE PROCESSING UNIT |
| 6 | INPUT UNIT |
| 7 | DISPLAY UNIT |
| 8 | STORAGE UNIT |
| 9 | CONTROL UNIT |
| 21 | ULTRASOUND TRANSDUCER |
| 31 | SIGNAL AMPLIFICATION UNIT |
| 41 | AMPLIFICATION CORRECTION UNIT |
| 42 | FREQUENCY ANALYSIS UNIT |
| 43 | FEATURE CALCULATION UNIT |
| 51 | B-MODE IMAGE DATA GENERATION UNIT |
| 52 | FEATURE IMAGE DATA GENERATION UNIT |
| 81 | FEATURE INFORMATION STORAGE UNIT |
| 101 | FEATURE IMAGE |
| 102 | SUPERPOSED IMAGE DISPLAY UNIT |
| 103 | INFORMATION DISPLAY UNIT |
| 431 | APPROXIMATION UNIT |
| 432 | ATTENUATION CORRECTION UNIT |
| 433 | OPTIMUM ATTENUATION RATE SETTING UNIT |
| $C_1$ | FREQUENCY SPECTRUM |

## Claims

1. An ultrasound observation apparatus comprising:

a frequency analysis unit configured to analyze a frequency of a signal generated based on an echo signal that is obtained by converting an ultrasound echo into an electric signal, the ultrasound echo having been generated from ultrasound transmitted to an observation target and reflected from the observation target, thereby to calculate a plurality of frequency spectra;
a feature calculation unit configured to:

calculate features of the plurality of frequency spectra;
perform attenuation correction for eliminating an effect of ultrasound attenuation on the features of the frequency spectra, for each of a plurality of attenuation rate candidate values giving different attenuation characteristics when the ultrasound propagates through the observation target, thereby to calculate corrected features of the frequency spectra; and
set an optimum attenuation rate for the observation target from among the plurality of attenuation rate candidate values, using the corrected features; and

a feature image data generation unit configured to generate feature image data for displaying the corrected features based on the optimum attenuation rate in association with visual information, together with an ultrasound image generated from the echo signal.

2. The ultrasound observation apparatus according to claim 1, wherein
the feature calculation unit is configured to:

approximate each of the frequency spectra by an nth-order expression (n is a positive integer) to calculate the features;
calculate statistical dispersion of the corrected features for each of the attenuation rate candidate values; and

set an attenuation rate candidate value having minimum statistical dispersion as the optimum attenuation rate.

3. The ultrasound observation apparatus according to claim 2, wherein
the feature calculation unit is configured to:

approximate a predetermined frequency band of the frequency spectra by a linear expression;
calculate, as the features, one or more of intercept of the linear expression, slope of the linear expression, and a mid-band fit, so as to include one of the slope and the mid-band fit, the mid-band fit being a value of the linear expression at an intermediate frequency of the frequency band; and
set the optimum attenuation rate based on one of the slope and the mid-band fit.

4. The ultrasound observation apparatus according to claim 3, wherein
the feature calculation unit is configured to:

set the optimum attenuation rate based on the slope if the slope is calculated as the features; and
set the optimum attenuation rate based on the mid-band fit if the mid-band fit is calculated as the features.

5. The ultrasound observation apparatus according to claim 2, wherein
the feature calculation unit is configured to:

calculate the statistical dispersion as a function of the attenuation rate candidate values; and
set the attenuation rate candidate value having the minimum statistical dispersion in the function, as the optimum attenuation rate.

6. The ultrasound observation apparatus according to claim 1, wherein
the feature calculation unit is configured to set the optimum attenuation rate for all frames of the ultrasound image.

7. The ultrasound observation apparatus according to claim 1, wherein
the feature calculation unit is configured to:

set the optimum attenuation rate every two or more predetermined number of frames of the ultrasound image; and
calculate the features of the frequency spectra, in a frame for which the optimum attenuation rate is not set, using the optimum attenuation rate that is lastly set previous to the frame.

8. The ultrasound observation apparatus according to claim 1, wherein
the feature calculation unit is configured to:

calculate an optimum attenuation rate equivalent value corresponding to the optimum attenuation rate for all frames of the ultrasound image; and
set the optimum attenuation rate based on the optimum attenuation rate equivalent value calculated for two or more predetermined number of frames.

9. The ultrasound observation apparatus according to claim 1, wherein
the feature image data contains information on the optimum attenuation rate.

10. The ultrasound observation apparatus according to claim 1, further comprising a display unit configured to display a feature image corresponding to the feature image data.

11. The ultrasound observation apparatus according to claim 1, further comprising an input unit configured to receive input for setting a target region for which the frequency spectra are calculated by the frequency analysis unit, wherein
the frequency analysis unit is configured to calculate the frequency spectra based on the ultrasound echo reflected from the target region.

12. The ultrasound observation apparatus according to claim 1, wherein
the feature calculation unit is configured to set the optimum attenuation rate using data with a dynamic range that is wider than a dynamic range of data used by the feature image data generation unit.

13. A method for operating an ultrasound observation apparatus, the method comprising:

a frequency analysis step of analyzing, by a frequency analysis unit, a frequency of a signal generated based on an echo signal that is obtained by converting an ultrasound echo into an electric signal, the ultrasound echo having been generated from ultrasound transmitted to an observation target and reflected from the observation target, thereby to calculate a plurality of frequency spectra;

a feature calculation step of calculating, by a feature calculation unit, features of the plurality of frequency spectra, performing attenuation correction for eliminating an effect of ultrasound attenuation on the features of the frequency spectra, for each of a plurality of attenuation rate candidate values giving different attenuation characteristics when the ultrasound propagates through the observation target, thereby to calculate corrected features of the frequency spectra, and setting an optimum attenuation rate for the observation target from among the plurality of attenuation rate candidate values, using the corrected features; and

a feature image data generation step of generating, by a feature image data generation unit, feature image data for displaying the corrected features based on the optimum attenuation rate in association with visual information, together with an ultrasound image generated from the echo signal.

14. A program for operating an ultrasound observation apparatus, the program causing the ultrasound observation apparatus to execute:

a frequency analysis step of analyzing, by a frequency analysis unit, a frequency of a signal generated based on an echo signal that is obtained by converting an ultrasound echo into an electric signal, the ultrasound echo having been generated from ultrasound transmitted to an observation target and reflected from the observation target, thereby to calculate a plurality of frequency spectra;

a feature calculation step of calculating, by a feature calculation unit, features of the plurality of frequency spectra, performing attenuation correction for eliminating an effect of ultrasound attenuation on the features of the frequency spectra, for each of a plurality of attenuation rate candidate values giving different attenuation characteristics when the ultrasound propagates through the observation target, thereby to calculate corrected features of the frequency spectra, and setting an optimum attenuation rate for the observation target from among the plurality of attenuation rate candidate values, using the corrected features; and

a feature image data generation step of generating, by a feature image data generation unit, feature image data for displaying the corrected features based on the optimum attenuation rate in association with visual information, together with an ultrasound image generated from the echo signal.

# FIG.1

# FIG.2

# FIG.3

# FIG.4

# FIG.5

# FIG.6

# FIG.7

# FIG.8

```
START
```

MEASURE NEW OBSERVATION TARGET — S1

AMPLIFY RECEIVED ECHO SIGNAL (STC CORRECTION) — S2

GENERATE B-MODE IMAGE DATA — S3

DISPLAY B-MODE IMAGE — S4

AMPLIFICATION CORRECTION — S5

FREQUENCY ANALYSIS — S6

CALCULATE PRE-CORRECTION FEATURE — S7

$\alpha = \alpha_0$ — S8

PERFORM ATTENUATION CORRECTION ON PRE-CORRECTION FEATURE USING ATTENUATION RATE CANDIDATE VALUE α — S9

CALCULATE VARIANCE OF CORRECTED FEATURE AFTER ATTENUATION CORRECTION — S10

$\alpha = \alpha + \Delta\alpha$ — S11

S12
$\alpha > \alpha_{max}$ ?   NO

YES

SET ATTENUATION RATE CANDIDATE VALUE HAVING MINIMUM VARIANCE OF CORRECTED FEATURE, AS OPTIMUM ATTENUATION RATE — S13

GENERATE FEATURE IMAGE DATA BASED ON OPTIMUM ATTENUATION RATE — S14

DISPLAY FEATURE IMAGE — S15

```
END
```

# FIG.9

FREQUENCY
ANALYSIS

$k=k_0$ — S21

$Z^{(k)}=Z^{(k)}_0$ — S22

OBTAIN AMPLITUDE DATA GROUP — S23

APPLY WINDOW FUNCTION — S24

IS AMPLITUDE DATA GROUP
NORMAL? — S25

NO

INSERT ZERO DATA TO
COVER SHORTFALL — S26

YES

FFT COMPUTATION — S27

$Z^{(k)}=Z^{(k)}+D$ — S28

$Z^{(k)}>Z^{(k)}_{max}$? — S29

NO

YES

$k=k+1$ — S30

NO

$k>k_{max}$? — S31

YES

RETURN

# FIG.10

S($\alpha$)

S($\alpha$)$_{min}$

0    0.2    0.4    0.6    0.8    1.0    $\alpha$ (dB/cm/MHz)

# FIG.11

102

101

ID:○○○○○

ATTENUATION RATE:

0.2(dB/cm/MHz)

103

# FIG.12

**INTERNATIONAL SEARCH REPORT**

| International application No. |
| --- |
| PCT/JP2015/060236 |

A. CLASSIFICATION OF SUBJECT MATTER
*A61B8/08*(2006.01)i

According to International Patent Classification (IPC) or to both national classification and IPC

B. FIELDS SEARCHED

Minimum documentation searched (classification system followed by classification symbols)
A61B8/08

Documentation searched other than minimum documentation to the extent that such documents are included in the fields searched

| Jitsuyo Shinan Koho | 1922–1996 | Jitsuyo Shinan Toroku Koho | 1996–2015 |
| Kokai Jitsuyo Shinan Koho | 1971–2015 | Toroku Jitsuyo Shinan Koho | 1994–2015 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
| --- | --- | --- |
| A | WO 2012/063976 A1 (Olympus Medical Systems Corp.),<br>18 May 2012 (18.05.2012),<br>entire text; all drawings<br>& JP 5054254 B2      & US 2012/0310087 A1<br>& EP 2548512 B1      & CN 102802536 A | 1–14 |
| A | WO 2013/179859 A1 (Olympus Medical Systems Corp.),<br>05 December 2013 (05.12.2013),<br>entire text; all drawings<br>& JP 5430809 B1      & US 2014/0099008 A1<br>& EP 2719337 A1      & CN 103717146 A | 1–14 |

☒   Further documents are listed in the continuation of Box C.     ☐   See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| 03 June 2015 (03.06.15) | 16 June 2015 (16.06.15) |

| Name and mailing address of the ISA/ | Authorized officer |
| --- | --- |
| Japan Patent Office<br>3-4-3,Kasumigaseki,Chiyoda-ku,<br>Tokyo 100-8915,Japan | |
| | Telephone No. |

Form PCT/ISA/210 (second sheet) (July 2009)

**INTERNATIONAL SEARCH REPORT**

| International application No. |
| --- |
| PCT/JP2015/060236 |

C (Continuation).    DOCUMENTS CONSIDERED TO BE RELEVANT

| Category* | Citation of document, with indication, where appropriate, of the relevant passages | Relevant to claim No. |
| --- | --- | --- |
| A | WO 2012/063978 A1  (Olympus Medical Systems Corp.), 18 May 2012 (18.05.2012), entire text; all drawings & JP 5054253 B2 & US 2012/0281497 A1 & EP 2548515 B1 & CN 102858251 A | 1-14 |

Form PCT/ISA/210 (continuation of second sheet) (July 2009)

**EP 3 167 809 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2008545123 T